# EUROPÄISCHE PATENTSCHRIFT

(11) **EP 0 410 246 B1**
(45) Veröffentlichungstag und Bekanntmachung des Hinweises auf die Patenterteilung: **29.12.1997**
(21) Anmeldenummer: 90113527.7
(22) Anmeldetag: 14.07.1990
(51) Int. Cl.: C12N 15/16, A61K 38/22, C12P 21/02, C12P 21/08, G01N 33/577, G01N 33/74, C07K 14/505

(54) **Erythropoietin (EPO)-Peptide und dagegen gerichtete Antikörper**
Erythropoietin(EPO)-peptides and antibodies against them
Peptides dérivés de l'érythropoiétine (EPO) et anticorps dirigés contre eux

(30) Priorität: 26.07.1989 DE 3924746
(43) Veröffentlichungstag der Anmeldung: 30.01.1991
(73) Patentinhaber: Behring Diagnostics GmbH, 35001 Marburg (DE)
(72) Erfinder: Fibi, Mathias, Dr., D-3550 Marburg (DE); Stüber, Werner, Dr., D-3551 Lahhntal (DE)

(56) Entgegenhaltungen:
- EP-A- 0 116 446
- WO-A-85/02610
- JOURNAL OF BIOLOGICAL CHEMISTRY. vol. 262, no. 3, 25 Januar 1987, BALTIMORE US Seiten 1161 - 1165; SYTKOWSKI, A.J. et al.: "Immunochemical studies of human erythropoietin using site-specific anti-peptide antibodies."
- Blood, vol.81, no.3, 1993, pp670-675, Fibi,M.R. et al.

## Beschreibung

Die Erfindung betrifft Erythropoietin (EPO)-Peptide und ihre Verwendung zur Herstellung von epitopspezifischen anti-EPO-Antikörpern. Ferner betrifft die Erfindung epitopspezifische anti-EPO-Antikörper, bei denen es sich um polyclonale Antikörper (Antiseren) oder um monoclonale Antikörper handeln kann. Außerdem betrifft die Erfindung die Verwendung der epitopspezifischen anti-EPO-Antikörper zur Reinigung von EPO, EPO-Derivaten oder EPO-Peptiden. Die Erfindung betrifft auch anti-idiotypische Antikörper, die einen Rezeptorbereich von EPO imitieren. Schließlich betrifft die Erfindung die Verwendung der epitopspezifischen anti-EPO-Antikörper zum Nachweis, vorzugweise zum epitopspezifischen Nachweis von EPO, Arzneimittel, die die genannten EPO-Peptide, anti-EPO-Antikörper oder anti-idiotypischen Antikörper enthalten, und Diagnostika zum Nachweis von EPO oder von anti-EPO-Antikörpern.

Erythropoietin (EPO) ist ein Glycoprotein-Hormon mit 166 Aminosäuren, 4 Glycosylierungsstellen an den AminosäurePositionen 24, 38, 83, 126 und einem Molekulargewicht von etwa 34 000. Es wird zunächst als Vorläuferprotein mit einem Signalpeptid von 23 Aminosäuren hergestellt. EPO stimuliert die mitotische Teilung und die Differenzierung von Erythrozyten-Vorläuferzellen und gewährleistet damit die Erythrozyten-Bildung. Es wird in der Niere produziert, wenn hypoxische Bedingungen herrschen. Im Rahmen einer EPO-induzierten Differenzierung von Erythrozyten-Vorläuferzellen wird die Globinsynthese induziert und die Synthese des Häm-Komplexes sowie die Anzahl der Ferritin-Rezeptoren steigen. Dadurch kann die Zelle mehr Eisen aufnehmen und funktionelles Hämoglobin synthetisieren: In den reifen Erythrozyten bindet Hämoglobin den Sauerstoff. Somit nehmen die Erythrozyten bzw. das in ihnen enthaltene Hämoglobin eine Schlüsselrolle für die Sauerstoffversorgung des Organismus ein. Ausgelöst werden die geschilderten komplexen Prozesse durch die Wechselwirkung von EPO mit einem entsprechenden Rezeptor auf der Zelloberfläche der Erythrozyten-Vorläuferzeller; vgl. Graber und Krantz, Ann. Rev. Med. 29 (1978), 51-66. EPO läßt sich entweder aus natürlichen Quellen, wie menschlichem Urin isolieren (vgl. z.B. Miyake et al., J. Biol. Chem. 252 (1977), 5558-5564) oder es kann durch gentechnologische Verfahren hergestellt werden (vgl. z.B. EP-A2 148 605).

Patienten mit Niereninsuffizienz können kein EPO bilden und leiden daher an einer Anämie. Versuche, diese EPO-Unterversorgung durch Verabfolgung von rekombinantem EPO zu komplettieren und die Symptome der Anämie zu verringern, sind bereits erfolgreich gewesen; vgl. The Lancet, 4. April 1987, "Erythropoietin", Seite 781-782; Eschbach et al., The New England Jorunal of Medicine 316 (1987), 73-78. Trotzdem ist über den Mechanismus der Wechselwirkung von EPO mit seinem Rezeptor noch wenig bekannt. Durch den Einsatz spezifischer Antikörper gegen EPO würde aber die Möglichkeit bestehen, das EPO-Molekül sowohl immunologisch als auch funktionell zu charakterisieren. Ferner könnten EPO-Regulationsstörungen mit neutralisierenden Antikörpern oder mit an den EPO-Rezeptor bindenden EPO-Peptiden behandelt werden. Dabei ist die Verwendung von EPO-Peptiden vorteilhaft gegenüber der Verwendung von vollständigen EPO-Molekülen, da sich Peptide leichter, z.B. auch synthetisch herstellen lassen.

EPO-Peptide, die den Positionen 1 bis 26, 40 bis 59, 80 bis 99, 99 bis 118, 111 bis 129, 131 bis 150 und 147 bis 166 entsprechen, sowie gegen einige dieser EPO-Peptide gerichtete Antikörper sind bereits aus Sytkowski und Donahue, J. Biol. Chem. 262 (1987), 1161-1165, bekannt. Antikörper, die die biologische Aktivität von EPO neutralisieren konnte aber von Sytkowski und Donahue nur mit EPO-Peptiden hergestellt werden, die den Positionen 99 bis 118 und 111 bis 129 entsprechen. Daraus schließen die Autoren, daß sich im Bereich der Aminosäure-Positionen 99 bis 129 von EPO die (einzige) Rezeptorbindungs-Domäne befindet. Dabei ist zu beachten, daß die EPO-Peptide zur Immunisierung über Glutardialdehydreste an einen Träger gebunden wurden.
In der EP-A2 148 605 werden neben der gentechnologischen Herstellung von EPO und Derivaten davon auch EPO-Peptide beschrieben, die den Positionen 1 bis 20, 41 bis 57, 116 bis 128 und 144 bis 166 beschrieben. Ferner werden polyclonale Kaninchen-Antikörper gegen diese EPO-Peptide beschrieben; vgl. EP-A2 148 605, Seite 90. Die Antikörper gegen das EPO-Peptid 116 bis 128 reagieren allerdings nicht mit EPO.
Neutralisierende Antikörper bzw. gegen Rezeptorbereiche von EPO gerichtete Antikörper werden in der EP-A2 148 605 nicht beschrieben.

Somit liegt der Erfindung das technische Problem zugrunde, neue EPO-Peptide bereitzustellen, die die weitere funktionelle und immunologische Charakterisierung von EPO gestatten. Ferner sollen neue EPO-Peptide bereitgestellt werden, die an den EPO-Rezeptor binden und damit zur Behandlung von EPO-Regulationsstörungen geeignet sind. Außerdem liegt der Erfindung das technische Problem zugrunde, Antikörper gegen die genannten EPO-Peptide bereitzustellen, die zum Nachweis von EPO und zur Behandlung von EPO-Funktionsstörungen geeignet sind.

Die Lösung des genannten technischen Problems wird durch die Bereitstellung der in den Patentansprüchen charakterisierten Ausführungsformen erzielt.

Gegenstand der Erfindung sind somit EPO-Peptide, die dadurch gekennzeichnet sind, daß sie die Aminosäurepositionen 1 bis 35 (P4), 142 bis 166 (P2) oder 152 bis 166 (P2/1) entsprechend der Numerierung der Aminosäurepositionen von natürlichem EPO aufweisen.

Die EPO-Peptide P2 und P2/1 gestatten überraschenderweise die Herstellung von neutralisierenden anti-EPO-Antikörpern. Erfindungsgemäß wird davon ausgegangen, daß diese EPO-Peptide einen weiteren, bisher noch nicht identifizierten Rezeptorbereich repräsentieren. Daher sind insbesondere diese erfindungsgemäßen EPO-Peptide zur Therapie von EPO-Regulationsstörungen geeignet.

Das EPO-Peptid P4 ist ebenfalls stark immunogen. Aufgrund der 100%igen Homologie von menschlichem EPO mit Affen- und Maus-EPO in diesem Bereich, sind Antikörper gegen diese erfindungemäßen EPO-Peptide nicht nur zum Nachweis von menschlichem EPO geeignet.

Insbesondere das Peptid P2 ist dem entsprechenden Epitop auf dem natürlich vorkommenden EPO-Molekül sehr ähnlich, denn mit diesem EPO-Peptid reagierende spezifische Antikörper sind zum Nachweis von natürlichem EPO beispielsweise im ELISA oder auch im Western-Blot geeignet.

Die erfindungsgemäßen Peptide lassen sich durch chemische oder enzymatische Spaltung von natürlischem EPO oder gentechnologisch gewonnenem EPO herstellen. Ferner können sie direkt gentechnologisch oder synthetisch hergestellt werden. Erfindungsgemäß wird die synthetische Herstellung bevorzugt.

Das erfindungsgemäße EPO-Peptid P4 hat die folgende Aminosäuresequenz:

P4 APPRLICDSRVLERYLLEAKEAENITTGCAEHCSL.

Die EPO-Peptide P2 und P2/1 haben die folgenden Aminosäure-Sequenzen:

P2 FRKLFRVYSNFLRGKLKLYTGEACRTGDR; oder
P2/1 KLKLYTGEACRTGDR.

Die erfindungsgemäßen EPO-Peptide lassen sich zur Herstellung von epitopspezifischen anti-EPO-Antikörpern verwenden. Diese Peptide bieten den Vorteil einer hochreinen validierbaren Substanz als Immunogen, das bei jeder Immunisierung reproduzierbar definierte, hochtitrige Antiseren induziert.

Mit den erfindungsgemäßen EPO-Peptiden lassen sich sowohl polyclonale epitopspezifische anti-EPO-Antikörper (Antiseren) als auch monoclonale epitopspezifische anti-EPO-Antikörper herstellen. Die Herstellung dieser Antikörper erfolgt in an sich bekannter Weise. Vorzugsweise werden die EPO-peptide jedoch erfindungsgemäß zur Immunisierung über Cysteinreste an ein Trägermaterial gebunden. Sofern die EPO-Peptide keinen Cysteinrest enthalten, wird ein solcher in üblicher Weise angefügt.

Die Erfindung betrifft ferner die mit den erfindungsgemäßen EPO-Peptiden herstellbaren epitopspezifischen anti-EPO-Antikörper. Vorteilhafterweise sind diese anti-EPO-Antikörper gegen bestimmte EPO-Epitope bzw. EPO-Domänen gerichtet. Dadurch können sie in Testsystemen eingesetzt werden, um normale EPO-Titer oder EPO-Titer während der Therapie epitopspezifisch nachzuweisen.

Die erfindungsgemäßen epitopspezifischen anti-EPO-Antikörper sind entweder polyclonale Antikörper (Antiseren) oder monoclonale Antikörper. Dadurch, daß die erfindungsgemäßen polyclonalen Antikörper mit den erfindungsgemäßen EPO-Peptiden hergestellt werden, ist ihre Herstellung reproduzierbar und sie sind definiert und hochtitrig. Deshalb eignen sie sich ganz besonders zum Einsatz in allen zu validierenden EPO-Nachweissystemen.

In einer anderen Ausführungsform betrifft die Erfindung anti-idiotypische Antikörper, die gegen die Bindungsregion der vorstehend genannten anti-EPO-Antikörper gerichtet sind und die einen EPO-Rezeptor-Bereich imitieren. Diese anti-idiotypischen Antikörper binden an den zellulären EPO-Rezeptor. Vorzugsweise sind diese anti-idiotypischen Antikörper gegen die Bindungsregion von anti-P2- oder anti-P2/1-Antikörper gerichtet.

In einer erfindungsgemäß bevorzugten Ausführungsform neutralisieren die epitopspezifischen anti-EPO-Antikörper die biologische Aktivität von EPO.

In einer weiteren bevorzugten Ausführungsform erkennen und beeinflussen die vorstehend genannten anti-idiotypischen Antikörper Zellen, die über EPO-Rezeptoren verfügen.

In einer anderen erfindungsgemäßen Ausführungsform werden die erfindungsgemäßen epitopspezifischen anti-EPO-Antikörper zur Reinigung von EPO, EPO-Derivaten oder EPO-Peptiden verwendet. Bei den entsprechenden Reinigungsverfahren handelt es sich um übliche chromatographische Verfahren, beispielsweise um Immunabsorptions-Chromatographien oder Affinitäts-Chromatographien. Zu diesem Zwecke werden die erfindungsgemäßen epitopspezifischen anti-EPO-Antikörper gegebenenfalls an zur Chromatographie geeignete Trägermaterialien gebunden.

Die Erfindung betrifft auch die Verwendung der erfindungsgemäßen epitopspezifischen anti-EPO-Antikörper zum Nachweis, vorzugsweise zum epitospezifischen Nachweis von EPO. Mit den Antikörpern lassen sich auch EPO-Muteine differentiell nachweisen. Solche EPO-Muteine können beispielweise in der Primärstruktur, d.h. in ihrer Aminosäure-Sequenz verändert sein. Spezifisch mit solchen EPO-Muteinen reagierende anti-EPO-Antikörper lassen sich unter Verwendung entsprechend angepaßter EPO-Peptide, wie sie vorstehend erläutert wurden, herstellen. Insbesondere betrifft die Erfindung Diagnostika, die die erfindungsgemäßen EPO-Peptide, epitopspezifischen anti-EPO-Antikörper und/oder anti-idiotypischen Antikörper enthalten.

In einer weiteren Ausführungsform betrifft die Erfindung Arzneimittel, die mindestens eines der erfindungsgemäßen EPO-Peptide oder erfindungsgemäße epitopspezifische anti-EPO-Antikörper enthalten. Vorzugsweise enthalten diese Arzneimittel EPO-Peptide, die zelluläre EPO-Rezeptoren blockieren. In einer anderen bevorzugten Ausführungsform enthalten die erfindungsgemäßen Arzneimittel erfindungsgemäße epitop-spezifische anti-EPO-Antikörper, die die biologische Aktivität von EPO neutralisieren. Gegebenenfalls enthalten die Arzneimittel ferner pharmazeutisch verträgliche Hilfs- und Zusatzstoffe.

Mit den genannten erfindungsgemäßen Arzneimitteln lassen sich EPO-Regulationsstörungen behandeln.

Die Figuren zeigen:

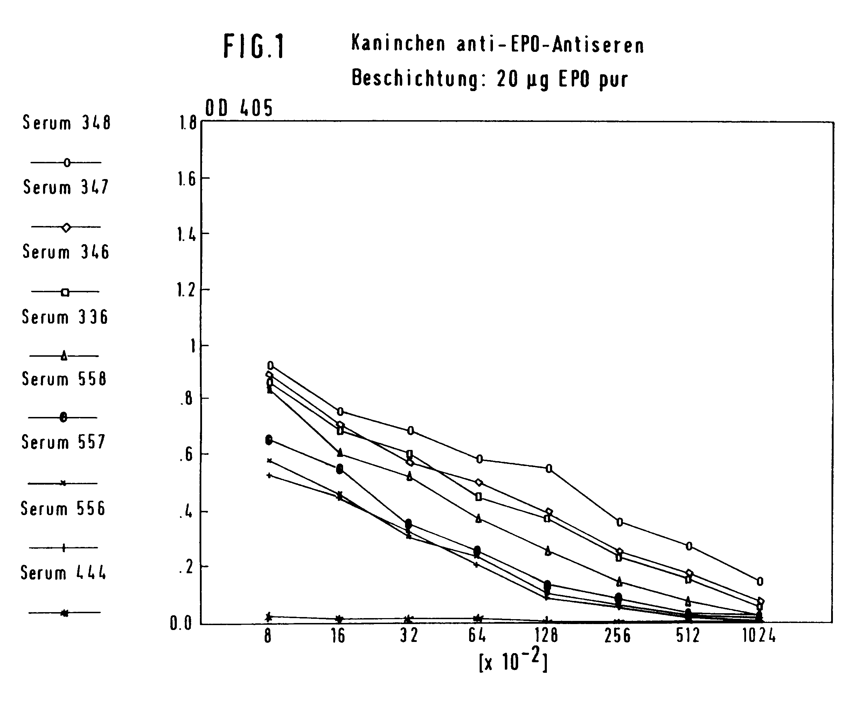

Fig. 1: Direkter Bindungstest an Erythropoetin. Serumverdünnungen von EPO-spezifischen bzw. P2/1-spezifischen Kaninchenantiseren wurden an EPO (20µg/ml)-beschichtete Mikrotiterplatten adsorbiert und gebundene Antikörper wurde mit enzymmarkierten anti-Kaninchen-Antikörpern nachgewiesen. Die Seren 336, 346, 347 und 348 sind gegen das EPO-Gesamtmolekül hergestellt, die Seren 556, 557, 558 sind gegen das Peptid P2 hergestellt, Serum 444 ist ein Kaninchenpräimmunserum.

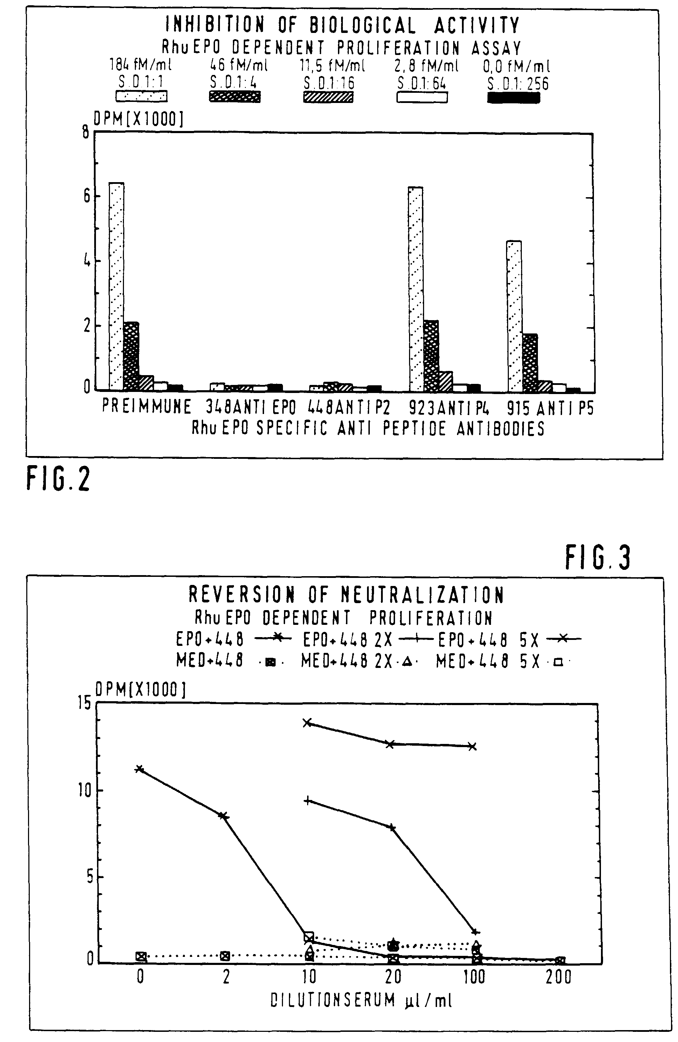

Fig. 2: Inhibition der biologischen Aktivität von rekombinantem humanem Erythropoetin (rhuEPO) durch rhuEPO spezifische Antiseren. Antiseren, die durch Immunisierung von Kaninchen mit rhuEPO oder den Peptidsequenzen P2, P4 und P5, gekoppelt an Keyhole limpet hemocyanin, gewonnen wurden, wurden zusammen mit rhuEPO in einem Proliferationstest von angereicherten erythroiden Vorläuferzellen nach Krystal (Exp. Hematol. 7, 649-660) eingesetzt. Antikörper, die durch Immunisierung mit P2-KLH oder EPO-KLH gewonnen wurden, inhibieren die durch rhuEPO induzierte Proliferation der erythroiden Vorläuferzellen. P5 hat die Peptidsequenz: LHVDKAVSGLRSLTTLLRALRAQKEAISPPDAA SAAPLRTITADT

Fig. 3: Reversion der Inhibition durch anti EPO-P2-KLH Seren. Vor der Inkubation der Vorläuferzellen mit rhuEPO und anti p2-KLH Antiserum im Krystal-Assay (s.o.), wurde das Serum an P2-Sepharose bis zu 5x präadsorbiert. Durch die Präadsorption an Peptid P2 läßt sich die inhibitorische Aktivität des Serums vollständig entfernen.

### Material und Methoden

### Herstellung der Peptide

Die Peptide wurden vorzugsweise nach der "solid phase"-Methode an einer Polystyrolmatrix hergestellt (1 % quervernetzt mit Di-vinylbenzol). Die Beladung der Polystyrolmatrix an funktionellen Gruppen (-NH₂) war vorzugsweise 0,4 - 0,6 mMol/g Matrix. Da die Peptide unter Verwendung der alkalilabilen Fmoc-Gruppe hergestellt wurden, wurden p-Alkoxybenzyl-Ester als Ankermoleküle verwendet. Im allgemeinen wurden während der Synthese Di-chlormethan und Dimethylformamid verwendet, in Ausnahmefällen N-Methylpyrrolidon. Die Menge der Wasch- oder Reaktionsflüssigkeiten betrugen vorzugsweise ca. 15 ml. Da die alpha-NH₂-Gruppen der Aminosäuren mit der Fmoc-Gruppe geschützt war, wurden folgende Schutzgruppen für die Seitengruppen der trifunktionellen Aminosäuren ausgewählt:

| | |
|---|---|
| Serin, Threonin, Tyrosin | tert.-Butyläther |
| Glutaminsäure, Asparaginsäure | tert.-Butylester |
| Arginin | 4-Methoxy-2,3,6-trimethylphenylsulfonyl |
| Cystein | tert.-Butylmercapto |
| Lysin | tert.-Butyloxycarbonyl |

Die Aminosäuren wurden vorzugsweise über Aktivester gekoppelt, besonders bevorzugt war eine in situ Aktivierung durch HOBt/Diisopropyl-carbodiimid.
Die repetitive alpha-NH₂-Schutzgruppenabspaltung erfolgte mit einer Base, vorzugsweise mit 20 % Piperidin in DMF bei Raumtemperatur.

Nach jedem dieser Reaktionsschritte, Kopplung bzw. Deblockierung, wurde das Harz mit DMF und Isopropylalkohol gewaschen.
Durch Acidolyse wurden simultan die Schutzgruppen von den Seitengruppen und die Peptide von der Matrix abgespalten. Vorzugsweise erfolgte dies mittels einer Mischung aus Trifluoressigsäure und Äthandithiol (9:1, v/v). Die Sulfhydrylgruppe der Cysteine wurde durch Thiolgruppen haltige Substanzen freigesetzt, wie beispielsweise Dithiothreitol oder Butylphosphin.

Die synthetischen Peptide wurden hinsichtlich ihrer chemischen Zusammensetzung und ihrer Reinheit untersucht. Die Zusammensetzung der Peptide wurde anhand einer Aminosäureanalyse bestimmt. Für diesen Zweck wurde eine kleine Probe mit 6N Salzsäure in Gegenwart von Phenol bei 110°C für 24 oder 72 Stunden hydrolysiert und die einzelnen Aminosäuren quantitativ bestimmt.
Die Peptidgehalte lagen bei etwa 85 %. Gegebenenfalls wurden die Peptide mittels RP-HPLC (reversed phase high performance liquid chromatography) nach bekannten Methoden gereinigt.
Die Reinheit der Peptide wurde durch HPLlC auf C18 reverse Phase Säulen bestimmt. Zu diesem Zweck wurde ein Phosphat/Acetonitril Gradient verwendet und es wurde eine Reinheit von mehr als 85 % gefunden.

### Kopplung der Peptide an Trägermoleküle

Die Peptide wurden vorzugsweise über Cysteinreste an ein hochmolekulares Trägerprotein, beispielsweise an Albumin oder Ovalbumin, vorzugsweise an Keyhole Limpet Hemocyanin (KLH) gekoppelt. Die Kopplung erfolgte über eine Thioätherbindung mit der Thiolgruppe des Cysteins. Diese Art der Kopplung hat den Vorteil, daß das Peptid in definierter Weise an das Trägerprotein gekoppelt wird. Sofern die Peptide keinen Cystein-Rest enthielten, wurde ein Cystein-Rest angehängt.

### Kopplung der Peptide an Sepharoseharze

Die Kopplung der Peptide an Sepharose erfolgte nach einem Standardverfahren mit Hilfe von Cyanbromid-aktivierter Sepharose.

### Herstellung der Antiseren

### Immunisierung von Kaninchen

Um EPO-Peptid-spezifische Antikörper zu produzieren, wurden die Peptid-KLH-Konjugate mit Adjuvantien emulgiert und gemäß eines 5-wöchigen Immunisierungsschemas in Kaninchen injiziert. Nach dieser Zeit hatten die Tiere spezifische Antikörper gebildet und wurden geblutet.

### Nachweis spezifischer Antikörper

Um die Seren auf Peptid-, bzw. Epo-Spezifität zu testen, wurde ein Festphasen ELISA verwendet. In Plastik-Mikrotiterplatten, die mit gereinigtem EPO oder mit EPO-Peptiden beschichtet waren, wurde der Gehalt an peptidspezifischen und EPO-spezifischen Antikörpern in den Antiseren bzw. in den affinitätschromatographisch gereinigten Antikörperfraktionen mit Hilfe eines enzymgekoppelten anti-Kaninchen-Ig-Antiserums bestimmt. Parallel dazu wurden die Antikörper im Western-Blot auf Nachweis der spezifischen EPO-Bande von 34-38 kDa überprüft.

### Nachweis neutralisierender Antikörper

Um die rhuEPO oder rhuEPO-Peptid spezifischen Antiseren auf neutralisierende Eigenschaften zu untersuchen, wurde der Proliferationstest nach Krystal (1983, Exp. Hematol. 7, 649 - 660) herangezogen. Weibliche NMRI-Mäuse wurden an zwei aufeinanderfolgenden Tagen mit 48 mg/kg Phenylhydrazin-hypochlorid injiziert. 48 Stunden nach der letzten Injektion wurden die Milzen der Tiere präpariert und eine Einzelzellsuspension hergestellt. Erythroide Vorläuferzellen wurden mit Hilfe eines Ficollgradienten (D = 1.077) angereichert. Für die Induktion der Proliferation dieser Zellen wurden in einer 96 Well Microtiterplatte 3 x 10 5 Zellen/ Well mit 0.1 pmol/ml rhuEPO inkubiert. Für die Durchführung der Inhibitionstests wurde EPO mit Verdünnungen der Antiseren vorinkubiert und dann im Proliferationstest eingesetzt.

Die Reinigung epitopspezifischer Antikörper erfolgte nach Standardmethoden, wobei die Antikörper aus einem gegen PBS dialysierten Ammoniumsulfat-Präzipitat über Nacht an die Sepharose adsorbiert wurde, ungebundenes Material mit PBS pH 7,0 heruntergewaschen wurde, und anschließend die spezifischen Antikörper mit saurem Wasser, pH 2,5, eluiert wurden. Die Eluate wurden sofort mit festem Natriumphosphat auf pH 7,0 neutralisiert und gegen PBS dialysiert.

### Kopplung epitopspezifischer Antikörper an Sepharoseharze

Die epitopspezifischen Antikörper wurden in einem Standardverfahren an Cyanbromid aktivierte Sepharose gekoppelt.

### Affinitätschromatographische Reinigung von Erythropoietin

Erythropoietin aus Zellkulturüberständen wurde an epitopspezifischen Antikörpersäulen adsorbiert und konnte ohne Verlust der biologischen Aktivität nach üblichen Verfahren durch einen Wechsel des pH-Wertes von pH 7 - 8 auf pH 2 - 3 eluiert werden.

Die Beispiele erläutern die Erfindung. Dabei werden die folgenden Abkürzungen verwendet:
- t-Bu: tert.-Butyläther
- Mtr: 4-Methoxy-2,3,6-trimethylphenylsulfonyl
- DMF: Dimethylformamid
- HOBt: Hydroxbenzotriazol
- Boc: tert.-Butyloxycarbonyl.
- Fmoc: Fluorenyloxycarbonyl
- GMBS: gamma-Maleimidobuttersäure-N-hydroxysuccinimidester

### Beispiel 1:

a) Peptidsynthese von (P2/1): 
H-Lys-Leu-Lys-Leu-Tyr-Thr-Gly-Glu-Ala-Cys-Arg-Thr-Gly-
Asp-Arg
   Die Synthese des Peptids erfolgte unter Verwendung eines vollautomatischen Peptidsynthesizers. Die Schutzgruppen wurdenvon lg Fmoc-Arg(Mtr)-p-alkoxybenzylesterharz mit 15 ml 20 % Piperidin / DMF (v/v) abgespalten und daraufhin wurde mit DMF und Isopropanol mehrfach gewaschen. Es wurden 1 mMol Fmoc-Asp(t-Bu)(dreifacher Überschuß) und 203 mg HOBt in 15 ml DMF gelöst zugesetzt. Nach Zugabe von 1,1 ml einer 1 ml Diisopropylcarbomidlösung (Dichlormethan) wurde die Kupplung während 1,5 Stunden ausgeführt. Durch Waschen mit DMF in Isopropanol wurden überschüssige Reagentien entfernt. Dieses Kupplungsschema wurde bis zur N-terminalen Aminosäure beibehalten. Als letzte Aminosäure wurde eine Boc-geschützte Aminosäure eingesetzt. Jeder Kupplungsschritt wurde durch einen Ninhydrintest auf Vollständigkeit geprüft. 1,06 g Harz wurden mit 2,5 ml Thianisol, 2,5 ml Äthandithiol und 15 ml Trifluoressigsäure 4 Stunden bei 35°C gerührt und abfiltriert. Die saure Lösung wurde in Äther gegossen, das ausgefällte Peptid abfiltriert und an einer Sephadex (R) G25 Säule 3 x 100 cm, 0,5 % Essigsäure chromatographiert. Der Peptidpool wurde lyophilisiert. Die Ausbeute betrug 230 mg.
b) Freisetzung der Sulfhydrylgruppe
   70 mg des Peptids wurden in 7 ml Trifluoräthanol und 350 µl Wasser gelöst und der pH-Wert mit N-Methylmorpholin auf 7,3 eingestellt. Das Reaktionsgefäß wurde mit Stickstoff gespült und mit 40 µl n-Tributylphosphin versetzt. Es wurde 1 Stunde bei Raumtemperatur gerührt, mit 50 ml Wasser verdünnt und der pH-Wert auf 4,0 eingestellt. Die Wasserphase wurde dreimal mit 10 ml Diäthyläther extrahiert, auf 10 ml ankonzentriert und an Sephadex^{R} G25 (3x 100 cm; 0,5 % Essigsäure) gereinigt. Nach Lyophilisierung erhielt man 55 mg Peptid.
c) Konjugatherstellung
   30 mg KLH (Keyhole-Limpet-Hemocyanin) wurden in 0,05 mMol Natriumphosphatpuffer, pH 8,0, gelöst und mit 3 mg GMBS 1 Stunde lang aktiviert. Das Protein wurde an einer Sephadex^{R} G50-Säule (2 x 30 cm) (0,1 Mol Natriumphosphat; 0,5 mMol EDTA, pH 6,0) chromatographiert. Der Proteinpool wurde auf 6 ml ankonzentriert und mit 30 mg des Sulfhydrylgruppen-haltigen Peptids 1 Stunde lang inkubiert. Nach Dialyse und Lyophilisierung wurden 38 mg Peptidkonjugat erhalten.

### Beispiel 2

### Immunisierung von Kaninchen

Kaninchen wurden mit jeweils 1,7 mg Antigen pro Tier über einen Zeitraum von 5 Wochen immunisiert. Bei der ersten Immunisierung erhielten die Tiere jeweils 0,4 mg Antigen in komplettem Freund's Adjuvans (CFA) Subkutan an 8 Immunisierungsstellen in der Nähe der Lymphknoten. Zwei Wochen später erfolgte eine subkutane Immunisierung mit 0,8 mg Antigen/Tier in CFA. Nach weiteren 2 Wochen wurden den Tieren an 5 Tagen hintereinander jeweils 0,1 mg Antigen in Aerosil intravenös verabreicht. Drei Tage später wurden sie geblutet und die einzelnen Antiseren gewonnen.

### Beispiel 3

### Herstellung von Immunadsorbentien mit Peptiden

Für die affinitätschromatographische Reinigung der Rohantiseren wurden ca. 20 mg beispielsweise des 15er Peptids (P2/1): 
H-Lys-Leu-Lys-Leu-Tyr-Thr-Gly-Glu-Ala-Cys-Arg-Thr-Gly-
Asp-Arg-OH
 an einer Festphase kovalent immobilisiert. Die Kupplungsreaktion erfolgte mit Bromcyan aktivierter Sepharose nach einem beschriebenen Verfahren (Axen et al., Nature 214 (1967), 1302). Anschließend wurde das Immunadsorbens jeweils mit phosphatgepufferter Kochsalzlösung (pBS; 0,15 Mol/l, pH 7,2) und Essigsäure (0,5 Mol/l, pH 2,5) gewaschen. Vor Verwendung wurde das Adsorbens mit dem dreifachen Volumen PBS äquilihbriert.
Ausbeute: ca. 20 ml Peptid - Sepharose.

Auf die gleiche Weise wurden die anderen Peptide zur Herstellung von Immunadsorbentien verwendet.

### Beispiel 4

### Gewinnung von Antikörpern

100 ml Rohantiserum wurden auf die mit PBS äquilibrierte Peptidsepharose (1,5 x 15 cm) aufgetragen und anschließend mit PBS gewaschen, bis die Extinktion bei 280 nm 0,01 betrug. Danach erfolgten Waschschritte mit 1 M NACl, pH 7,0, und Wasser (pH 7,0), wobei jeweils das 3-fache Gelvolumen verwendet wurde. Die Elution der Antikörper von Immunadsorbens erfolgte mit Wasser (pH 2,5), die Antikörperlösung wurde mit festem Natriumphosphat (0,001 Mol/l) auf pH 7,0 eingestellt, ankonzentriert (Amicon-Membran) und bei -70°C gelagert. Ausbeute: 35mg Antikörper.

### Beispiel 5

### Austesten der Antikörper

a) Herstellung von EPO- bzw. Peptid-beschichteten Mikrotiterplatten
   In Plastik Mikrotiterplatten wurden 20 µg/ml EPO bzw. EPO-spezifische Peptide über Nacht bei 4°C in Carbonatpuffer gekoppelt. Vor Durchführung der Tests wurden die Platten zweimal mit PBS gewaschen, mit PBS 0,5 % BSA für 30 Minuten abgesättigt und anschließend dreimal mit PBS gewaschen.
b) Durchführung der Enzymimmunoassays
   Die mit BSA abgesättigten Platten wurden 2 Stunden mit Verdünnungen der anti-EPO-Kaninchenantiseren bzw. der gereinigten Antikörperfraktionen inkubiert. Nach dieser Zeit wurde mit PBS gewaschen und 2 Stunden mit anti-Kaninchen-Ig-Antiserum inkubiert, welches mit alkalischer Phosphatase gekoppelt war. Danach wurde zweimal mit PBS gewaschen, dann zweimal mit 0,2 M Tris-HCl, pH 9,5, und danach kurz mit 1 M Tris-HCl, pH 9,5. Nach einer Stunde wurde die Reaktion mit 1 M NaOH gestoppt und die optische Dichte bei 405 nm gemessen.
c) Durchführung von Western-Blots
   EPO-Standards wurden durch Polyacrylamid-Gelelektrophorese aufgetrennt und auf Nitrocellulose übertragen (Towbin et al., Proc. Natl. Acad. Sci. USA, 76 (1979), 4350 - 4354). Die Filter wurden in PBS, 0,5 % BSA abgesättigt und danach mit den Antikörpern über Nacht inkubiert. Danach wurden die Filter dreimal mit PBS gewaschen und mit alkalischer Phosphatase konjugiertem anti-Kaninchen-Ig-Antiserum 2 Stunden inkubiert. Nach dreimaligem Waschen in PBS, einmaligem Waschen in 0,2 M Tris-HCl, pH 9,5 und kurzem Waschen in 1 M Tris-HCl, pH 9,5, wurden die Blots mit 4-Nitrotetrazolium-chloridblau-hydrat (500 µg/ml) und 5-Brom-4-chlorindoxylphospat-p-toluidiniumsalz (200 µg/ml) in 1 M Tris-HCl, pH 9,5, entwickelt. Nach 20 Minuten wurde die Reaktion mit Wasser gestoppt.

### Beispiel 6

### Herstellung von Immunadsorbentien mit Antikörpern

20 bis 50 mg affinitätschromatographisch gereinigte Antikörper wurden nach einem Standardverfahren (Axen et al., Nature 214 (1967), 1302) an Bromcyan-aktivierte Sepharose gekoppelt und wie in Beispiel 3 beschrieben weiterbehandelt.

### Beispiel 7

### Affinitätschromatographische Reinigung von Erythropoietin

Immunadsorbentien, die EPO-spezifische Antikörper enthielten, wurden wie vorstehend beschrieben zur affinitätschromatographischen Reinigung von EPO und EPO-Muteinen herangezogen.

### Beispiel 8

### Herstellung monoklonaler Antikörper

Zur Herstellung monoklonaler Antikörper wurden rekombinant gewonnenes EPO sowie die oben beschriebenen Peptide als Antigen eingesetzt. Die Peptide wurden zuvor an KLH (Keyhole limpet haemocyanin) gekoppelt.

Balb/c Mäuse (weibl.) wurden intraperitoneal bzw. subcutan mit 10 µg immunisiert, und über mehrere Wochen geboostert. Unmittelbar vor der eigentlichen Fusion wurden die Versuchstiere zusätzlich 4 Tage hintereinander intravenös geboostert.

Am Tage der Fusion wurden die Milzen steril entnommen und zu Einzelzellen suspendiert. Durch Fusion von 10⁸ Milzzellen mit 2 x 10⁷ Zellen einer Myelomzellinie (SP 2/0) wurden Hybridzellen erzeugt, welche anschließend in einem Selektionsmedium DMEM (Dulbecco's minimal essential Medium) +20 % FKS (fetales Kälberserum); 0,1 mM Hypoxanthin; 0,4 mM Aminopterin, 16 mM Thymidin) auf 24-Well Platten (Costar), in einer Konzentration von 10⁶ Zellen/Well, ausgesät wurden. 2 - 3 Wochen später wurden aus den Wells einzelne Zellkolonien isoliert und in jeweils ein anderes Well neuer Kulturplatten (24-Well, Costar) übertragen. Nach weiteren 2 - 3 Tagen wurden diese Kulturüberstände in einem Enzymimmunoassay auf das Vorhandensein von Anti-EPO-Antikörpern gescreent. Spezifische Antikörper produzierende Hybride wurden selektioniert und mit Hilfe eines Single-Cell-Manipulators kloniert.

### Beispiel 9

### Herstellung und Nachweis Anti-idiotypischer Antikörper

Für die Immunisierung wurde ein synogener monoklonaler Antikörper mit gewünschter Spezifität gegen EPO/EPO-Peptid eingesetzt. Anstelle des Ganzantikörpers wurde das Fab'-Fragment an BSA oder KLH gekoppelt und weiblichen Balb/c Mäusen intraperitoneal bzw. subcutan in CFA (komplettes Freund'sches Adjuvants) injiziert. Die weitere Herstellung der monoklonalen anti-idiotypischen Antikörper entspricht dem in Beispiel 8 beschriebenen Verfahren.

Kulturüberstände wurden in einem Enzymimmunoassay, unter Verwendung des Immunisierungsantikörpers konjugiert mit Peroxidase (POD), auf vorhandene anti-Idiotyp Antikörper und diese wiederum in einem weiteren Enzymimmunoassay auf ihre Antigenhemmbarkeit überprüft. Antigenhemmbare anti-Idiotyp Antikörper produzierende Hybride wurden selektioniert und mit Hilfe eines Single-Cell-Manipulators kloniert.

## Patentansprüche (Patentansprüche für folgende(n) Vertragsstaat(en): AT, BE, CH, DE, DK, FR, GB, IT, LI, LU, NL, SE)

1. Immunogene Erythropoietin (EPO)-Peptide, mit einer der folgenden Aminosäuresequenzen:

P4 APPRLICDSRVLERYLLEAKEAENITTGCAEHCSL;
P2 FRKLFRVYSNFLRGKLKLYTGEACRTGDR; oder
P2/1 KLKLYTGEACRTGDR

2. Verfahren zur Herstellung der EPO-Peptide nach Anspruch 1, dadurch gekennzeichnet, daß man
a) die entsprechenden, jeweils geschützten Aminosäuren an einer Matrix schrittweise koppelt und nach dem letzten Reaktionsschritt die Schutzgruppen von den Seitenketten und die Peptide von der Matrix abspaltet; oder
b) die Peptide gentechnologisch herstellt; oder
c) natürliches oder gentechnologisch gewonnenes EPO chemisch oder enzymatisch spaltet und die genannten Peptide isoliert.

3. Verwendung von EPO-Peptiden nach Anspruch 1 zur Herstellung von epitopspezifischen neutralisierenden anti-EPO-Antikörpern, wobei sich ein Epitop definitionsgemäß aus einem oder mehreren Peptiden, oder aus einer oder mehreren Peptidteilsequenzen zusammensetzen kann.

4. Verwendung nach Anspruch 3, dadurch gekennzeichnet, daß die Antikörper monoclonal sind.

5. Epitopspezifische anti-EPO-Antikörper, dadurch gekennzeichnet, daß sie gegen ein EPO-Peptid nach Anspruch 1 gerichtet sind oder nach dem Verfahren gemäß Anspruch 2 hergestellt worden sind und die biologische Aktivität von EPO neutralisieren.

6. Epitopspezifische anti-EPO-Antikörper nach Anspruch 5, dadurch gekennzeichnet, daß sie monclonale Antikörper sind.

7. Anti-idiotypische Antikörper gegen die Bindungsregion von Antikörpern nach Anspruch 5.

8. Verwendung der Antikörper nach Anspruch 5 zur Reinigung von EPO, EPO-Derivaten oder EPO-Peptiden.

9. Diagnostikum enthaltend Antikörper nach Anspruch 5 zum Nachweis von EPO.

10. Diagnostikum enthaltend EPO-Peptide nach Anspruch 1 zum Nachweis von anti-EPO-Antikörpern.

11. Arzneimittel, enthaltend mindestens ein EPO-Peptid nach Anspruch 1 oder Antikörper nach Anspruch 5 und gegebenenfalls pharmazeutisch verträgliche Hilfs- und Zusatzstoffe.

12. Arzneimittel, enthaltend anti-idiotypische Antikörper nach Anspruch 7 und gegebenenfalls pharmazeutisch verträgliche Hilfs- und Zusatzstoffe.

13. Arzneimittel nach Anspruch 11 oder 12 zur Behandlung von Regulationsstörungen der Hämatopoese.

14. Diagnostikum, enthaltend anti-idiotypische Antikörper nach Anspruch 7 zum Nachweis von neutralisierenden Antikörpern oder EPO-Rezeptoren.

## Patentansprüche (Patentansprüche für folgende(n) Vertragsstaat(en): ES)

1. Verfahren zur Herstellung von immunogenen Erythropoietin (EPO)-Peptiden, mit einer der folgenden Aminosäuresequenzen: 
P4 APPRLICDSRVLERYLLEAKEAENITTGCAEHCSL;
P2 FRKLFRVYSNFLRGKLKLYTGEACRTGDR; oder
P2/1 KLKLYTGEACRTGDR,
 wobei man
a) die entsprechenden, jeweils geschützten Aminosäuren an einer Matrix schrittweise koppelt und nach dem letzten Reaktionsschritt die Schutzgruppen von den Seitenketten und die Peptide von der Matrix abspaltet; oder
b) die Peptide gentechnologisch herstellt; oder
c) natürliches oder gentechnologisch gewonnenes EPO chemisch oder enzymatisch spaltet und die genannten Peptide isoliert.

2. Verfahren zur Herstellung von epitopspezifischen anti-EPO-Antikörpern, welche die biologische Aktivität von EPO neutralisieren, dadurch gekennzeichnet, daß man die nach Anspruch 1 erhaltenen EPO-Peptide an ein Trägerprotein kuppelt, mit dem erhaltenen Konjugat Säuger immunisiert und die Antikörper in an sich bekannter Weise gewinnt.

3. Verfahren nach Anspruch 2, dadurch gekennzeichnet, daß man polyclonale Antikörper aus dem Serum der immunisierten Säuger gewinnt.

4. Verfahren nach Anspruch 3, dadurch gekennzeichnet, daß man zur Herstellung monoclonaler Anitkörper Milzzellen der immunisierten Säuger mit Myelomzellen fusioniert und die von den erhaltenen Hybridomazellen produzierten monoclonalen Antikörper gewinnt.

5. Verfahren nach einem der Ansprüche 2 bis 4, dadurch gekennzeichent, daß die erhaltenen epitopspezifischen anti-EPO-Antikörper die biologische Aktivität von EPO neutralisieren.

6. Verfahren zur Herstellung von anti-idiotypischen Antikörpern gegen die Bindungsregion von gemäß Anspruch 5 erhaltenen EPO-neutralisierenden Antikörpern, dadurch gekennzeichnet, daß man Säuger mit diesen Antikörpern immunisiert und aus deren Serum die anti-idiotypischen Antikörper gewinnt.

7. Verfahren zur Reinigung von EPO, EPO-Derivaten oder EPO-Peptiden, dadurch gekennzeichnet, daß man die nach einem der Ansprüche 2 bis 5 erhaltenen epitopspezifischen anti-EPO-Antikörper an zur Chromatographie geeignete Trägermaterialien bindet, die zu reinigenden Substanzen an den Träger adsorbiert und eluiert.

## Patentansprüche (Patentansprüche für folgende(n) Vertragsstaat(en): GR)

1. Immunogene Erythropoietin (EPO)-Peptide, mit einer der folgenden Aminosäuresequenzen:

P4 APPRLICDSRVLERYLLEAKEAENITTGCAEHCSL;
P2 FRKLFRVYSNFLRGKLKLYTGEACRTGDR; oder
P2/1 KLKLYTGEACRTGDR

2. Verfahren zur Herstellung der EPO-Peptide nach Anspruch 1, dadurch gekennzeichnet, daß man
a) die entsprechenden, jeweils geschützten Aminosäuren an einer Matrix schrittweise koppelt und nach dem letzten Reaktionsschritt die Schutzgruppen von den Seitenketten und die Peptide von der Matrix abspaltet; oder
b) die Peptide gentechnologisch herstellt; oder
c) natürliches oder gentechnologisch gewonnenes EPO chemisch oder enzymatisch spaltet und die genannten Peptide isoliert.

3. Verwendung von EPO-Peptiden nach Anspruch 1 zur Herstellung von epitopspezifischen neutralisierenden anti-EPO-Antikörpern, wobei sich ein Epitop definitionsgemäß aus einem oder mehreren Peptiden, oder aus einer oder mehreren Peptidteilsequenzen zusammensetzen kann.

4. Verwendung nach Anspruch 3, dadurch gekennzeichnet, daß die Antikörper monoclonal sind.

5. Epitopspezifische anti-EPO-Antikörper, dadurch gekennzeichnet, daß sie gegen ein EPO-Peptid nach Anspruch 1 gerichtet sind oder nach dem Verfahren gemäß Anspruch 2 hergestellt worden sind und die biologische Aktivität von EPO neutralisieren.

6. Epitopspezifische anti-EPO-Antikörper nach Anspruch 5, dadurch gekennzeichnet, daß sie monclonale Antikörper sind.

7. Anti-idiotypische Antikörper gegen die Bindungsregion von Antikörpern nach Anspruch 5.

8. Verwendung der Antikörper nach Anspruch 5 zur Reinigung von EPO, EPO-Derivaten oder EPO-Peptiden.

9. Verwendung der epitopspezifischen anti-EPO-Antikörper nach Anspruch 5 oder 6 zur Herstellung eines Diagnostikums zum Nachweis von EPO.

10. Verwendung der EPO-Peptide nach Anspruch 1 zur Herstellung eines Diagnostikums zum Nachweis von anti-EPO-Antikörpern.

11. Verwendung mindestens eines der EPO-Peptide nach Anspruch 1 oder eines epitopspezifischen anti-EPO-Antikörpers nach Anspruch 5 oder 6 zur Herstellung eines Arzneimittels zur Behandlung von Regulationsstörungen der Hämatopoese.

12. Verwendung eines anti-idiotypischen Antikörpers nach Anspruch 7 zur Herstellung eines Arzneimittels zur Behandlung von Regulationsstörungen der Hämatopoese.

13. Verwendung eines anti-idiotypischen Antikörpers nach Anspruch 7 zur Herstellung eines Diagnostikums zum Nachweis von neutralisierenden Antikörpern oder EPO-Rezeptoren.

## Claims (Claims for the following Contracting State(s): AT, BE, CH, DE, DK, FR, GB, IT, LI, LU, NL, SE)

1. An immunogenic erythropoietin (EPO) peptide having one of the following amino-acid sequences:

P4        APPRLICDSRVLERYLLEAKEAENITTGCAEHCSL;
P2        FRKLFRVYSNFLRGKLKLYTGEACRTGDR; or
P2/1      KLKLYTGEACRTGDR

2. A process for preparing an EPO peptide as claimed in claim 1, which comprises
a) stepwise coupling of the appropriate amino acids, protected in each case, to a matrix and, after the last reaction step, eliminating the protective groups from the side chains and the peptide from the matrix; or
b) preparing the peptide by genetic manipulation; or
c) cleaving, chemically or enzymatically, natural or genetically engineered EPO, and isolating said peptide.

3. The use of an EPO peptide as claimed in claim 1 for the preparation of epitope-specific neutralizing anti-EPO antibodies, it being possible for an epitope by definition to consist of one or more peptides or one or more sections of peptide sequences.

4. The use as claimed in claim 3, wherein the antibodies are monoclonal.

5. An epitope-specific anti-EPO antibody, which is directed against an EPO peptide as claimed in claim 1 or has been prepared by the process claimed in claim 2 and which neutralizes the biological activity of EPO.

6. An epitope-specific anti-EPO antibody as claimed in claim 5, which is a monoclonal antibody.

7. An anti-idiotype antibody against the binding region of an antibody as claimed in claim 5.

8. The use of an antibody as claimed in claim 5 for purifying EPO, EPO derivatives or EPO peptides.

9. A diagnostic aid comprising an antibody as claimed in claim 5 for detecting EPO.

10. A diagnostic aid comprising an EPO peptide as claimed in claim 1 for detecting anti-EPO antibodies.

11. A pharmaceutical comprising at least one EPO peptide as claimed in claim 1 or antibody as claimed in claim 5 and, where appropriate, pharmaceutically suitable auxiliaries and additives.

12. A pharmaceutical comprising an anti-idiotype antibody as claimed in claim 7 and, where appropriate, pharmaceutically suitable auxiliaries and additives.

13. A pharmaceutical as claimed in claim 11 or 12 for the treatment of disturbances of haematopoiesis regulation.

14. A diagnostic aid comprising an anti-idiotype antibody as claimed in claim 7 for the detection of neutralizing antibodies or EPO receptors.

## Claims (Claims for the following Contracting State(s): ES)

1. A process for the preparation of immunogenic erythropoietin (EPO) peptides having one of the following amino-acid sequences: 
P4          APPRLICDSRVLERYLLEAKEAENITTGCAEHCSL;
P2          FRKLFRVYSNFLRGKLKLYTGEACRTGDR; or
P2/1        KLKLYTGEACRTGDR
 which comprises
a) stepwise coupling of the appropriate amino acids, protected in each case, to a matrix and, after the last reaction step, eliminating the protective groups from the side chains and the peptide from the matrix; or
b) preparing the peptide by genetic manipulation; or
c) cleaving, chemically or enzymatically, natural or genetically engineered EPO, and isolating said peptide.

2. A process for the preparation of epitope-specific anti-EPO antibodies which neutralize the biological activity of EPO, which comprises coupling the EPO peptides obtained as claimed in claim 1 to a carrier protein, immunizing mammals with the resulting conjugate, and isolating the antibodies in a manner known per se.

3. The process as claimed in claim 2, wherein polyclonal antibodies are isolated from the serum of the immunized mammals.

4. The process as claimed in claim 3, wherein to prepare monoclonal antibodies spleen cells of the immunized mammals are fused to myeloma cells, and the monoclonals antibodies produced by the resulting hybridomacells are isolated.

5. The process as claimed in any of claims 2 to 4, wherein the resulting epitope-specific anti-EPO antibodies neutralize the biological activity of EPO.

6. A process for the preparation of anti-idiotype antibodies against the binding region of EPO-neutralizing antibodies obtained as claimed in claim 5, which comprises immunizing mammals with these antibodies, and isolating the anti-idiotype antibodies from their serum.

7. A process for the purification of EPO, EPO derivatives or EPO peptides, which comprises binding the epitope-specific anti-EPO antibodies obtained as claimed in any of claims 2 to 5 to support materials suitable for chromatography, and adsorbing the substances to be purified on the support and eluting them.

## Claims (Claims for the following Contracting State(s): GR)

1. An immunogenic erythropoietin (EPO) peptide having one of the following amino-acid sequences:

P4        APPRLICDSRVLERYLLEAKEAENITTGCAEHCSL;
P2        FRKLFRVYSNFLRGKLKLYTGEACRTGDR; or
P2/1      KLKLYTGEACRTGDR

2. A process for preparing an EPO peptide as claimed in claim 1, which comprises
a) stepwise coupling of the appropriate amino acids, protected in each case, to a matrix and, after the last reaction step, eliminating the protective groups from the side chains and the peptide from the matrix; or
b) preparing the peptide by genetic manipulation; or
c) cleaving, chemically or enzymatically, natural or genetically engineered EPO, and isolating said peptide.

3. The use of an EPO peptide as claimed in claim 1 for the preparation of epitope-specific neutralizing anti-EPO antibodies, it being possible for an epitope by definition to consist of one or more peptides or one or more sections of peptide sequences.

4. The use as claimed in claim 3, wherein the antibodies are monoclonal.

5. An epitope-specific anti-EPO antibody, which is directed against an EPO peptide as claimed in claim 1 or has been prepared by the process claimed in claim 2 and which neutralizes the biological activity of EPO.

6. An epitope-specific anti-EPO antibody as claimed in claim 5, which is a monoclonal antibody.

7. An anti-idiotype antibody against the binding region of an antibody as claimed in claim 5.

8. The use of an antibody as claimed in claim 5 for purifying EPO, EPO derivatives or EPO peptides.

9. The use of an epitope-specific anti-EPO antibody as claimed in claim 5 or 6 for the preparation of a diagnostic aid for the detection of EPO.

10. The use of an EPO peptide as claimed in claim 1 for the preparation of a diagnostic aid for the detection of anti-EPO antibodies.

11. The use of at least one EPO peptide as claimed in claim 1 or of an epitope-specific anti-EPO antibody as claimed in claim 5 or 6 for the preparation of a pharmaceutical for the treatment of disturbances of haematopoiesis regulation.

12. The use of an anti-idiotype antibody as claimed in claim 7 for the preparation of a pharmaceutical for the treatment of disturbances of haematopoiesis regulation.

13. The use of an anti-idiotype antibody as claimed in claim 7 for the preparation of a diagnostic aid for the detection of neutralizing antibodies or EPO receptors.

## Revendications (Revendications pour l'(les) Etat(s) contractant(s) suivant(s): AT, BE, CH, DE, DK, FR, GB, IT, LI, LU, NL, SE)

1. Peptides d'érythropoïétine (EPO) immunogènes, comportant l'une des séquences d'aminoacides suivantes:

P4   APPRLICDSRVLERYLLEAKEAENITTGCAEHCSL,
P2   FRKLFRVYSNFLRGKLKLYTGEACRTGDR, ou
P2/1 KLKLYTGEACRTGDR.

2. Procédé pour la préparation des peptides d'EPO selon la revendication 1, caractérisé en ce que
a) on couple par étapes les aminoacides correspondants, chacun protégé, à une matière de support, et, après la dertrière étape de réaction, on sépare les groupes protecteurs des chaînes latérales et on sépare les peptides de la matière de support; ou
b) on prépare les peptides par génie génétique; ou
c) on coupe par voie chimique ou enzymatique de l'EPO naturelle ou obtenue par génie génétique, et on isole lesdits peptides.

3. Utilisation de peptides d'EPO selon la revendication 1, pour la production d'anticorps neutralisants anti-EPO spécifiques d'épitopes, un épitope selon la définition pouvant se composer d'un ou de plusieurs peptides ou d'une ou de plusieurs séquences partielles de peptides.

4. Utilisation selon la revendication 3, caractérisée en ce que les anticorps sont monoclonaux.

5. Anticorps anti-EPO spécifiques d'épitopes, caractérisés en ce qu'ils sont dirigés contre un peptide d'EPO. selon la revendication 1 ou ont été produits selon le procédé conforme à la revendication 2, et neutralisent l'activité biologique de l'EPO.

6. Anticorps anti-EPO spécifiques d'épitopes selon la revendication 5, caractérisés en ce qu'ils sont des anticorps monoclonaux.

7. Anticorps anti-idiotypiques dirigés contre la région de fixation d'anticorps selon la revendication 5.

8. Utilisation des anticorps selon la revendication 5, pour la purification d'EPO, de dérivés d'EPC ou de peptides d'EPO.

9. Agent de diagnostic contenant des anticorps selon la revendication 5, pour la détection de l'EPO.

10. Agent de diagnostic contenant des peptides d'EPO selon la revendication 1, pour la détection d'anticorps anti-EPO.

11. Médicament, contenant au moins un peptide d'EPO selon la revendication 1 ou un anticorps selon la revendication 5 et éventuellement des additifs et adjuvants pharmaceutiquement acceptables.

12. Médicament, contenant des anticorps anti-idiotypiques selon la revendication 7 et éventuellement des adjuvants et additifs pharmaceutiquement acceptables.

13. Médicament selon la revendication 11 ou 12, destiné au traitement de troubles de la régulation de l'hématopoïèse.

14. Agent de diagnostic, contenant des anticorps anti-idiotypiques selon la revendication 7, pour la détection d'anticorps neutralisants ou de récepteurs d'EPO.

## Revendications (Revendications pour l'(les) Etat(s) contractant(s) suivant(s): ES)

1. Procédé pour la préparation de peptides d'érythropoïétine (EPO) immunogènes, comportant l'une des séquences d'aminoacides suivantes: 
P4 APPRLICDSRVLERYLLEAKEAENITTGCAEHCSL,
P2 FRKLFRVYSNFLRGKLKLYTGEACRTGDR, ou
P2/1 KLKLYTGEACRTGDR,
 dans lequel
a) on couple par étapes les aminoacides correspondants, chacun protégé, à une matière de support, et, après la dernière étape de réaction, on sépare les groupes protecteurs des chaînes latérales et on sépare les peptides de la matière de support; ou
b) on prépare les peptides par génie génétique; ou
c) on coupe par voie chimique ou enzymatique de l'EPO naturelle ou obtenue par génie génétique, et on isole lesdits peptides.

2. Procédé pour la production d'anticorps anti-EPO spécifiques d'épitopes, qui neutralisent l'activité biologique de l'EPO, caractérisé en ce l'on couple à une protéine porteuse les peptides d'EPO obtenus selon la revendication 1, on immunise des mammifères avec le congugué obtenu et on obtient les anticorps d'une façon connue en soi.

3. Procédé selon la revendication 2, caractérisé en ce que l'on obtient des anticorps polyclonaux à partir du sérum des mammifères immunisés.

4. Procédé selon la revendication 3, caractérisé en ce que, pour la production d'anticorps monoclonaux, on fait fusionner des cellules spléniques des mammifères immunisés avec des cellules de myélome, et on obtient les anticorps monoclonaux produits par les hybridomes obtenus.

5. Procédé selon l'une des revendications 2 à 4, caractérisé en ce que les anticorps anti-EPO spécifiques d'épitopes obtenus neutralisent l'activité biologique de l'EPO.

6. Procédé pour la production d'anticorps anti-idiotypiques dirigés contre la région de fixation d'anticorps neutralisant l'EPO, obtenus selon la revendication 5, caractérisé en ce que l'on immunise des mammifères avec des anticorps et on obtient les anticorps anti-idiotypiques à partir de leur sérum.

7. Procédé pour la purification d'EPO, de dérivés d'EPO ou de peptides d'EPO, caractérisé en ce que l'on fixe à des matériaux de support appropriés à la chromatographie les anticorps anti-EPO spécifiques d'épitopes, obtenus selon l'une des revendications 2 à 5, et les substances à purifier sont adsorbées sur le support et éluées.

## Revendications (Revendications pour l'(les) Etat(s) contractant(s) suivant(s): GR)

1. Peptides d'érythropoïétine (EPO) immunogènes, comportant l'une des séquences d'aminoacides suivantes:

P4 APPRLICDSRVLERYLLEAKEAENITTGCAEHCSL,
P2 FRKLFRVYSNFLRGKLKLYTGEACRTGDR, ou
P2/1 KLKLYTGEACRTGDR.

2. Procédé pour la préparation des peptides d'EPO selon la revendication 1, caractérisé en ce que
a) on couple par étapes les aminoacides correspondants, chacun protégé, à une matière de support, et, après la dernière étape de réaction, on sépare les groupes protecteurs des chaînes latérales et on sépare les peptides de la matière de support; ou
b) on prépare les peptides par génie génétique; ou
c) on coupe par voie chimique ou enzymatique de l'EPO naturelle ou obtenue par génie génétique, et on isole lesdits peptides.

3. Utilisation de peptides d'EPO selon la revendication 1, pour la production d'anticorps neutralisants anti-EPO spécifiques d'épitopes, un épitope selon la définition pouvant se composer d'un ou de plusieurs peptides ou d'une ou de plusieurs séquences partielles de peptides.

4. Utilisation selon la revendication 3, caractérisée en ce que les anticorps sont monoclonaux.

5. Anticorps anti-EPO spécifiques d'épitopes, caractérisés en ce qu'ils sont dirigés contre un peptide d'EPO selon la revendication 1 ou ont été produits selon le procédé conforme à la revendication 2, et neutralisent l'activité biologique de l'EPO.

6. Anticorps anti-EPO spécifiques d'épitopes selon la revendication 5, caractérisés en ce qu'ils sont des anticorps monoclonaux.

7. Anticorps anti-idiotypiques dirigés contre la région de fixation d'anticorps selon la revendication 5.

8. Utilisation des anticorps selon la revendication 5, pour la purification d'EPO, de dérivés d'EPO ou de peptides d'EPO.

9. Utilisation des anticorps anti-EPO spécifiques d'épitopes selon la revendication 5 ou 6, pour la préparation d'un agent de diagnostic destiné à la détection de l'EPO.

10. Utilisation des peptides d'EPO selon la revendication 1, pour la préparation d'un agent de diagnostic destiné à la détection d'anticorps anti-EPO

11. Utilisation d'au moins l'un des peptides d'EPO selon la revendication 1 ou d'un anticorps anti-EPO spécifique d'épitope selon la revendication 5 ou 6, pour la fabrication d'un médicament destiné au traitement de troubles de la régulation de l'hématopoïèse.

12. Utilisation d'un anticorps anti-idiotypique selon la revendication 7, pour la fabrication d'un médicament destiné au traitement de troubles de la régulation de l'hématopoïèse.

13. Utilisation d'un anticorps anti-idiotypique selon la revendication 7, pour la fabrication d'un agent de diagnostic destiné à la détection d'anticorps neutralisants ou de récepteurs d'EPO.
